# EUROPEAN PATENT SPECIFICATION

(11) **EP 2 872 135 B1**
(45) Date of publication and mention of the grant of the patent: **26.10.2016**
(21) Application number: 13736751.2
(22) Date of filing: 04.07.2013
(51) Int. Cl.: A61K 31/375, A61K 36/738, A61P 19/02

(54) **PROCESS FOR THE MANUFACTURE OF ROSE HIP POWDER**
VERFAHREN ZUR HERSTELLUNG VON HAGEBUTTENPULVER
PROCÉDÉ POUR LA FABRICATION DE LA POUDRE DE CYNORRHODON

(30) Priority: 06.07.2012 DK 201270413; 06.07.2012 US 201261668751 P
(43) Date of publication of application: 20.05.2015
(73) Proprietor: Orkla Health A/S, 2635 Ishøj (DK)
(72) Inventor: BRUSTAD, Irena, N-1386 Asker (NO); HELLAND, Anita, N-2830 Raufoss (NO); SLEE, Emily Louise, Bulli, NSW 2516 (AU); PINSTRUP, Martin Stadil, DK-4000 Roskilde (DK); HØEG, Johannes Arbo, N-0256 Oslo (NO)
(74) Representative: Protector IP Consultants AS
(86) International application number: PCT/DK2013/050224
(87) International publication number: WO 2014/005597

(56) References cited:
- WO-A1-99/53934
- WO-A1-2008/006582
- WO-A1-2010/048955
- WO-A2-2008/006589
- WILLICH S N ET AL: "Rose hip herbal remedy in patients with rheumatoid arthritis a randomised controlled trial", PHYTOMEDICINE, GUSTAV FISCHER VERLAG, STUTTGART, DE, vol. 17, no. 2, February 2010 (2010-02), pages 87-93, XP026832079, ISSN: 0944-7113 [retrieved on 2009-10-08]
- JOSEPH SCHWAGER ET AL: "Rose hip and its constituent galactolipids confer cartilage protection by modulating cytokine, and chemokine expression", BMC COMPLEMENTARY AND ALTERNATIVE MEDICINE, BIOMED CENTRAL LTD., LONDON, GB, vol. 11, no. 1, 3 November 2011 (2011-11-03), page 105, XP021111918, ISSN: 1472-6882, DOI: 10.1186/1472-6882-11-105
- WINTHER K ET AL: "A powder made from seeds and shells of a rose-hip subspecies (Rosa canina) reduces symptoms of knee and hip osteoarthritis: a randomized, double-blind, placebo-controlled clinical trial", SCANDINAVIAN JOURNAL OF RHEUMATOLOGY, ALMQVIST & WIKSELL PERIODICAL CO., STOCKHOLM, SE, vol. 34, no. 4, August 2005 (2005-08), pages 302-308, XP009171045, ISSN: 0300-9742
- Anon: "Litomove", Avellus A/S , 7 March 2012 (2012-03-07), XP002712109, Retrieved from the Internet: URL:http://web.archive.org/web/20120307194 426/http://www.axellus.dk/c-52-LITOMOVE.as px [retrieved on 2013-08-29]

## Description

The present disclosure relates to a composition comprising dried rose hip and vitamin C, and a process for obtaining said rose hip composition. It also relates to the use of said composition in a method for the maintenance of flexible joints and decreased inflammation. Furthermore, it also relates to a complex and the use of said complex and the composition for the treatment of inflammatory diseases, in particular osteoarthritis.

The invention is defined in the claims.

### Background

Degenerative arthritis refers to a clinical syndrome in which low-grade inflammation results in pain in the joints, caused by abnormal wearing of the cartilage that covers and acts as a cushion inside joints and destruction or decrease of synovial fluid that lubricates those joints.

No curative treatment is presently available, however treatment with anti-inflammatory agents have shown effective in e.g. alleviating pain with patients suffering from degenerative arthritis.

Rose hip formulations are known to have anti-inflammatory properties and have also been shown effective in the treatment of the above-mentioned symptoms. US 6,024,960 describes a rose hip formulation as an anti-inflammatory agent for alleviating/reducing symptoms associated with inflammation and arthritis. The formulation is prepared from rose hips in a process comprising the steps of harvesting rose hips when the hips are fully ripe, chopping the harvested rose hips into pieces, drying the chopped rose hips to a water content of at most 5% by weight, passing the dried and chopped rose hips through a separation step in which nuts, hairs and other extraneous matter is removed, and crushing the remaining material in a grinding mill to a particle size of below 1 mm such as about 0.1 to 0.5 mm. The step of drying the chopped rose hips is conducted at a temperature below 50°C with air and by avoiding sun light in order to preserve the vitamin content of the rose hips. The resultant powder according to US 6,024,960 comprises several vitamins such as vitamin A, vitamin B, vitamin C, vitamin E and vitamin K as well as several minerals, in particular the rose hip powder contains a high content of vitamin B, E and C with about 560 mg vitamin C per 100 g powder in addition to a wide range of minerals.

WO 2008/006589 also describes an anti-inflammatory composition comprising rose hips prepared according to the process of US 6,024,960 and at least one additional component.

According to WO 2008/006589 the prepared rose hips should preferably at least comprise glycosides of diacylglycerol such as 3-beta-D-galactopyranosyloxy-2-(octadeca-9Z,12Z,15Z-trienoyloxy) propanyl octadeca-9Z,12Z,15Z-trienoate and a high content of vitamin C, which may be in the range of 0.6 to 1.5 mg pr g. The additional components are suggested as being ascorbyl phosphate and lycopene.

At present it is not known which specific ingredients in rose hip provide the anti-inflammatory effects as well as the positive results in the treatment and alleviation of pain in the patients. Thus, it is difficult to identify the optimal conditions for providing the rose hip composition, or preparing compositions providing the desired effect and as a result often a large dosage of the rose hip compositions must be administered to a patient in order to achieve the desired anti-inflammatory effect.

An amount of 4500 mg of the rose hip powder obtainable from the process according to US 6,024,960 is suggested for providing an anti-inflammatory effect. Administering such dose typically requires ingesting amount of pills, tablets or capsules, such as 6 capsules a day. Patients in general, however, prefer to take a reduced amount of pharmaceuticals and nutraceuticals, thus making it desirable to reduce the amount of rose hip required to provide an anti-inflammatory effect.

Therefore, it is an object of the present invention to provide an improved rose hip composition, being improved in the effect and concentration of effective ingredients and patient compliance.

### Summary

With this background, it is an object of the present disclosure in a first aspect to provide a composition comprising dried rose hip powder and added vitamin C, wherein the dried rose hip powder is obtainable by a process comprising the steps of providing harvested rose hips, drying and chopping the rose hips, separating the chopped rose hips and isolating the shells, grinding the shells, and optionally sieving the ground rose hip shells to a particle size below 1 mm, wherein the grinding is performed at a substantially constant temperature, said temperature being below ambient temperature, such as below 40°C, preferably below 30°C, more preferred below 25°C even more preferred below 20°C.

The composition is characterised by comprising a high concentration of active ingredients. The present inventors have identified these active ingredients as comprising vitamin C, carotenes, flavonoids, triterpenoic acids, and galactolipids. Accordingly, in an embodiment of the present invention the composition obtainable by the process is characterised in that it comprises vitamin C, carotenes, flavonoids, triterpenoic acids, and galactolipids. Said active ingredients of the composition may also be denoted by the trademark name ROSENOIDS® and/or ROSENOIDS® complex. The composition is in its broadest sense characterised in having an anti arthritic effect which is double the effect of prior art products, as can be seen from example 2. Therefore, in an embodiment of the present invention the composition is used in a method for the maintenance of flexible joints and decreased inflammation. The present inventors attribute the improved anti-arthritic effect to the high concentrations of the active ingredients obtainable by the process according to the present invention.

Furthermore, the composition has an improved shelf live meaning that the effect is less susceptible to degradation than those of the prior art.

The present inventors have further found that the composition according to the invention contains a reduced amount of triacylglycerols (TAGs) and their fragments as compared to rose hip compositions obtained by conventional methods. The reduced amount of TAGs may be attributed to the isolation of the shells as they are believed to contain a reduced amount of TAGs as compared to other parts of rose hips, i.a. the seeds and fruit flesh. TAGs may be linked to atherosclerosis and, by extension, the risk of heart disease and stroke.

Without the wish to be bound by any theory it is presently believed that it is not the drying process which is critical to the preservation of active ingredients as believed in the prior art methods but rather the separation of shells from other constituents and the grinding at cool temperatures. It was found that the grinding process develops heat and since the grinding provides open cells of the rose hip shells the active ingredients within the cells are more susceptible to degradation than during the drying process.

Clinical studies performed with the composition according to the present invention have demonstrated that said composition provides a beneficial effect in the treatment of inflammatory diseases, in particular in the treatment of osteoarthritis. The composition is at least as good as an equivalent composition comprising dried rose hips provided by conventional processing. In particular, the studies have shown that the composition according to the present invention is at least as good as an equivalent composition comprising dried rose hips provided by conventional processing even in the low dose, said low dose being half that of the prior art.

It is believed that the dried rose hip composition obtainable by the process accounts for the observed increased inflammatory effects by an increased concentration of the active ingredients. Consequently, a subject in need of the treatment or preventative treatment can ingest a smaller dosage in order to obtain the same effect of treatment. With the composition the administration dosage required for obtaining an anti-inflammatory effect in a patient is 2250 mg. Accordingly, only 3 standard capsules a day is required in order to obtain an inflammatory effect. Thus, it may increase patient compliance.

The composition further comprises added vitamin C. Rose hips are particularly high in vitamin C content, thus the total content of vitamin C may partly be from the rose hip shells themselves. However, an additional amount of vitamin C is added to the composition. The presence of vitamin C is believed to facilitate the uptake in a subject of the active ingredients of the rose hip complex.

In a preferred embodiment the vitamin C is a synthetic vitamin as naturally occurring vitamin C in rosehip has shown to degrade too fast. Added vitamin C has been found to improve the stability of the composition in addition to the stability provided by the natural occurring vitamin C of the rose hip powder.

The amount of vitamin C may be added in an amount of from about 30 mg added vitamin C /g rose hip powder to about 80 mg added vitamin C /g rose hip powder. In a preferred embodiment vitamin C is added to an amount of about 80 mg to about 2250 mg of rose hip powder. The vitamin C may be a synthetic vitamin C. The additional vitamin C is further believed to provide for improved stability of the composition, thus improving the shelf-life of the product.

In a preferred embodiment the composition is obtained from rose hip. However, the present inventors envisage that the specific concentrations of the individual active ingredients of the composition may also be provided by alternative means such as addition and/or supplement of the individual active ingredients.

By the term high concentration of active ingredients is meant that the composition comprises vitamin C in an amount of from about 3.2 to about 11.2 g/kg, carotenes in an amount of from about 150 to about 560 mg/kg, flavonoids in an amount of from about 100 to about 1300 mg/kg, triterpenoic acids in an amount of from about 500 to about 2000 mg/kg, and galactolipids in an amount of from about 50 to about 500 mg/kg. The high amount of active ingredients may be obtained according to the process of the present invention.

Therefore, another aspect of the present invention relates to a process for the manufacture of a rose hip composition comprising the steps of (a) providing harvested rose hip, (b) drying and chopping the rose hip, (c) separating the chopped rose hip and isolating the shells, (d) grinding the shells, and (e) optionally sieving the ground rose hip shells to a particle size below 1 mm, wherein the grinding is performed at a constant temperature, said temperature being below ambient temperature, such as below 40°C, preferably below 30°C, more preferred below 25°C even more preferred below 20°C.

The step of grinding typically produces heat due to friction and the heat causes increased temperature of the shells subject to grinding. Thus, the components of the rose hips are subject to the increase temperature, which is believed to result in the degradation of the active constituents of the rose hip complex. Therefore, the process step of grinding the shells must be conducted in a careful manner so as not to increase the temperature above this maximum temperature.

The present inventors have developed specific settings and parameters for a grinding mill to comply with these conditions. Thus, the grinding is performed at a constant temperature, said temperature being below ambient temperature so that the active ingredients of the complex are not destroyed by e.g. denaturation. The ambient temperature may be 40°C, 30°C, 29°C, 28°C, 27°C, 26°C, 25°C, 24°C, 23°C, 22°C, 21°C, or 20°C. In a preferred embodiment of the present invention the temperature is below 28°C, more preferably below 25°C, more preferably below 23°C, and even more preferably below 20°C. In another embodiment the temperature is in the range of 10-40°C, such as 12-28°C, 14-26°C, 15-25°C, 15-23°C, 15-22°C, 16-21°C, 18-20°C or 19-20°C. In a particular embodiment cooling is applied during the step of grinding in order to maintain the substantially constant temperature being below ambient temperature. In a preferred embodiment the cooling applied is air cooling.

The present inventors have shown that the rose hip powder obtained according to the process comprises the high concentration of the active ingredients as defined for the composition in the first aspect of the present invention. The present inventors have analysed the contents of the rose hip powder by metabolomic profiling and found that rose hip powder obtained from the shells of rose hip, which are ground at a constant temperature, said temperature being below ambient temperature, comprises a higher concentration of the active ingredients, i.e. vitamin C, carotenes, flavonoids, triterpenoic acids, and galactolipids, as compared to rose hip powder obtained by conventional means.

Being that rose hip is a natural product the resulting composition of the dried rose hip may vary accordingly. The present inventors have found that the composition obtainable by the process may further comprise minute to no remnants of tocopherols and α-linolenic acid, such as substantially no amount of tocopherols and α-linolenic acid. The present inventors have shown that the composition according to the invention comprising vitamin C, carotenes, flavonoids, triterpenoic acids, and galactolipids, and minute to substantially no amount of tocopherols and α-linolenic acid provides increased anti-inflammatory effects. Accordingly, the composition obtainable by the process comprising vitamin C, carotenes, flavonoids, triterpenoic acids, and galactolipids has shown effective for the maintenance of flexible joints and decreased inflammation. An even further effect may be obtained if tocopherols and α-linolenic acid are added to the composition; such further effect may be directed to the above-mentioned decreased inflammation. Such further effect may also relate to other beneficial properties provided by tocopherols and α-linolenic acid. In an embodiment of the present invention the natural amounts of tocopherols and α-linolenic resulting from the process is maintained.

The present inventors found that the above concentrations of the active ingredients may be provided by using the shells of the rose hips. More preferred by grinding the shells of the rosehips at a constant temperature below ambient temperatures, most preferred below 25°C, even more preferred below 20°C.

In yet another embodiment the process further comprises the step of mixing the ground rose hip with vitamin C to provide a composition of ground rosehip powder and vitamin C.

Both the compositions and the rose hip powder obtained from either of the first two aspects are in another aspect for use as a medicament and/or nutraceutical.

Also the compositions and the rose hip powders are for use in a method for treating and/or prevention of inflammatory diseases or the rose hip powders may be used in a method for the maintenance of flexible joints and decreased inflammation. Said compositions and rose hip powder may also be for use in a method for treating arthritis, more preferred degenerative arthritis.

Also provided is the use of any of the compositions and the rose hip powder for the manufacture of a medicament for use in the treatment and/or prevention of inflammatory diseases, in particular arthritis and more specifically degenerative arthritis. Another embodiment relates to the use of any of the compositions and the rose hip powder for the manufacture of a medicament and/or nutraceutical for use in a method for the maintenance of flexible joints and decreased inflammation.

In another aspect a complex at least comprising vitamin C, carotenes, tocopherols, flavonoids, triterpenoic acids, α-linolenic acid, and galactolipids is provided. The present inventors have found that a combination of these constituents has proven that patients suffering from osteoarthritis when treated with the complex comprising the individual constituents showed significant pain relief.

In a preferred embodiment the complex at least comprises vitamin C in an amount of from about 3.2 to about 11.2 g/kg, carotenes in an amount of from about 150 to about 560 mg/kg, tocopherols in an amount of from about 120 to about 250 mg/kg, flavonoids in an amount of from about 500 to about 1300 mg/kg, triterpenoic acids in an amount of from about 500 to about 1060 mg/kg, α-linolenic acid in an amount of from about 0.15 to about 2.2 g/kg, and galactolipids in an amount of from about 85 to about 1030 mg/kg.

When present in these ranges the anti-inflammatory effect is believed to be effective so that the daily dosage can be halved without affecting the anti-inflammatory effect. Thus, it was found that with the ranges given above administering more of the complex did not result in an increased anti-inflammatory effect.

In an embodiment the complex is for use as a medicament and/or nutraceutical, and in a preferred embodiment the complex is for use in the treatment and/or prevention of inflammatory diseases. Thus, the present disclosure also relates to the use of the complex for the manufacture of a medicament for the treatment and/or prevention of inflammatory diseases. In another preferred embodiment the inflammatory disease is arthritis, more preferred degenerative arthritis.

In a preferred embodiment the complex is obtained from rose hip and even more preferred from rose hip according to any of the aspects of the invention.

Thus, the specific concentrations of the individual constituents of the complex may be provided by use of the process according to the present invention or by alternative means such as addition of the individual constituents. In particular, the composition obtainable by the process according to the present invention may be added additional amounts of the individual constituents, such as tocopherols and/or α-linolenic acid.

### Figures

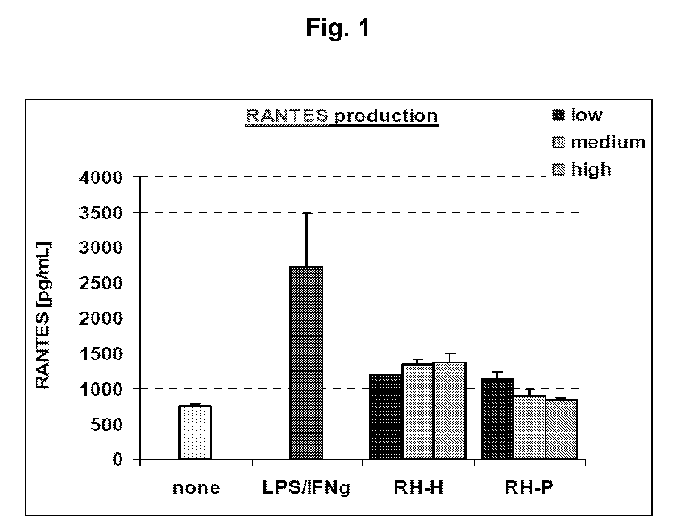

Figure 1 shows the results of an in vitro study of the effects of two rose hip compositions (RH-H and RH-P) on the protein secretion of CCL5/RANTES by peripheral blood leukocytes (PBL) in an induced inflammatory response. The protein secretion is presented for PBL incubated for 24 h at the indicated conditions: non-stimulated (none); activated with endotoxin (lipopolysaccharide, LPS) and IFN-γ to induce an inflammatory response (LPS/IFNg); activated with LPS/IFNγ in the presence of 125 mg/L (low, left column), 250 mg/L (medium, middle column) and 500 mg/L (high, right column) of RH-H or RH-P, respectively.
Figure 2 shows the results of an in vitro study of the effects of two rose hip compositions (RH-H and RH-P) on the protein secretion of CXCL10/IP-10 by PBLs in an induced inflammatory response. The protein secretion is presented for PBL incubated for 24 h at the indicated conditions: non-stimulated (none); activated with LPS/IFN-γ (LPS/IFNg); activated with LPS/IFNγ in the presence of 125 mg/L (low, left column), 250 mg/L (medium, middle column) and 500 mg/L (high, right column) of RH-H or RH-P, respectively.

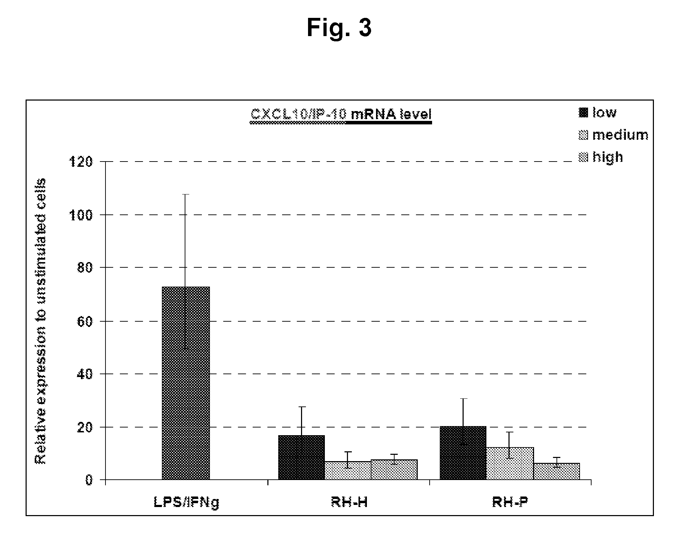

Figure 3 shows the results of an in vitro study of the effects of two rose hip compositions (RH-H and RH-P) on the gene expression of CXCL10/IP-10 by PBL in an induced inflammatory response. The protein secretion is presented for PBL incubated for 12 h at the indicated conditions: non-stimulated (none); activated with LPS/IFN-γ to induce an inflammatory response (LPS/IFNg); activated with LPS/IFNγ in the presence of 125 mg/L (low, left column), 250 mg/L (medium, middle column) and 500 mg/L (high, right column) of RH-H or RH-P, respectively. The relative gene expression is expressed as fold change (*versus* unstimulated cells, arbitrarily set at 1).
Figure 4 shows a comparison of different preparations and doses of rose hip powder in patients with osteoarthritis of the knee as detailed in example 2. These amounts of the individual constituents of the rose hip complex are found to exceed the average concentrations contained in rose hips. Therefore, it is suggested that the above-mentioned complex having the individual constituents in these concentrations provides an increased potency with respect to anti-inflammatory properties.
Figure 5 shows DART-TOFMS spectra of individual samples thereby providing a comparison of metabolomic profiles for MeOH/H₂O extracts from four different rose hip powder samples derived from Rosa canina, Rosa moschata, and Rosa rubiginosa (positive ion mode, 250°C). Samples A and C are manufactured by conventional processes, whereas samples B and D are obtained by the process according to the present invention.
Figure 6 shows DART-TOFMS spectra of samples A to D for a metabolomic profile comparison of ethyl acetate extracts of the samples (positive ion mode, 250°C).
Figure 7 shows DART-TOFMS spectra of samples A to D for a metabolomic profile comparison of hexane extracts of the samples (positive ion mode, 250°C).
Figure 8 shows DART-TOFMS spectra of samples A to D for a metabolomic profile comparison of MeOH/H₂O extracts of the samples (negative ion mode, 250°C).
Figure 9 shows DART-TOFMS spectra of samples A to D for a metabolomic profile comparison of ethyl acetate extracts of the samples (negative ion mode, 250°C).
Figure 10 shows DART-TOFMS spectra of samples A to D for a metabolomic profile comparison of hexane extracts of the samples (negative ion mode, 250°C).
Figure 11 shows a graphical comparison of the flavonoid content in the rose hip powder samples A to D. The flavonoids were determined using ultra performance liquid chromatography coupled with tandem mass spectrometer. The results are also summarised in Table 7.

### Detailed description of the invention

In the following the present invention is described in more detail. All features and details can equally be applied to any of the composition, complex, and process.

In the first aspect of the present disclosure relates to a composition comprising dried rose hip and vitamin C, wherein the dried rose hip is obtainable by a process comprising the steps of providing harvested rose hips, drying and chopping the rose hips, separating the chopped rose hips and isolating the shells, grinding the shells, and optionally sieving the ground rose hip to a particle size below 1 mm, wherein the grinding is performed at a constant temperature, said temperature being below ambient temperature, such as 40°C.

The rose hips may be obtained from any one of the species of plants belonging to the Rosa family. As rose hip is a natural product the composition of the dried rose hip may vary accordingly. The process for obtaining the dried rose hip should not be construed as limiting the scope of the invention. The dried rose hip may be obtained by any alternative way of processing. However, the dried rose hip of the first aspect of the invention shows a greater effect than prior art products because the active ingredients of rose hip are present in an increased concentration compared to conventional dried rose hip. In particular, the dried rose hip obtainable by said process contains an increased amount of active ingredients denoted as the ROSENOIDS® and/or ROSENOIDS® complex, which is believed to comprise active ingredients of rose hips for the treatment and prevention of a variety of conditions, such as inflammatory diseases.

By the term rose hip is meant the fruit from the rose plant. The rose hips may be obtained from any one of the species of plants belonging to the Rosa family such as Rosa canina, Rosa mochata, Rosa rubiginosa, Rosa gallica, Rosa conditata, Rosa rugosa, Rosa hugonis, Rosa nitida, Rosa pendulina, Rosa pimpinellifolia and Rosa sericea as well as any mixture thereof. The present invention should, however, not be limited to these rose hips or rose hips from any particular species of rose plants.

Rose hip is a natural product and therefore various environmental factors influence the outcome of the crops. Such variations may be due to but not limited to soil nutritional conditions across fields, weather conditions during the cultivation season such as average temperature and rainfall, pests, harvesting and other environmental influences such as fertilisation. Hence, the composition of the dried rose hip may vary accordingly. The content of the dried rose hip may also vary between the species of rose hip. The fruit from the rose plant is typically red-to-orange and consists of a shell that encloses clusters of seeds surrounded by fruit flesh.

Providing the harvested rose hip implies that the rose hips are harvested when the fruit is matured or fully riped. The harvesting process is common general knowledge to the person skilled in the art. The additional steps of drying and chopping the rose hips as well as separating and isolating the shells may also be done using techniques well known in the art. For example as described in US 6024960 wherein the drying is performed at a temperature under 50°C using air and avoiding sunlight. The drying is however not limited to this method. Any suitable drying technique may be used.

After the drying the rose hips are chopped. After the chopping the rosehip is separated from various extraneous materials.

The chopping and separation may be performed in any suitable means which is well known in the art,

Further, the inventors have found that an improved composition is obtained when only the shells of the dried rose hips are used. In a preferred embodiment the dried rose hips substantially consist of the shells from the rose hips. Without being bound by any specific theory it is believed that the shells comprise an increased amount of the active ingredients, i.e. vitamin C, carotenes, flavonoids, triterpenoic acids, and galactolipids, as compared to the other constituents of rose hip, e.g. the seeds and pulp. Therefore, providing the composition with the processed shells increases the concentration of the active ingredients in the composition. In order to retain maximum of activity of active ingredients of the dried rose hips the temperature of the rose hips should not exceed ambient temperature during processing. The active ingredients are found to be more vulnerable during the grinding as compared to the drying. Thus, in the drying process step the harvested rose hips may exceed the temperatures of the grinding step, and should preferably but not necessarily comply with the requirement as described in the prior art.

In an embodiment the composition comprises dried rose hip and vitamin C, wherein the dried rose hip is obtained by the process according to another aspect of the invention. In this aspect of the present invention the process for the manufacture of dried rose hip comprises the steps of (a) providing harvested rose hips, (b) drying and chopping the rose hips, (c) separating the chopped rose hips and isolating the shells, (d) grinding the shells, and optionally (e) sieving the ground rose hip to a particle size below a suitable particle size, such as below 1 mm, wherein the grinding is performed at a constant temperature, said temperature being below ambient temperature, such as below 40°C, preferably below 30°C, more preferred below 25°C and even more preferred below 20°C. Accordingly, in an embodiment the temperature is below 40°C.

Initially, harvested rose hips are provided, which implies that the rose hips are harvested when the fruit is matured or fully riped, thus ensuring that the active ingredients are present. The harvesting process is common general knowledge with the person skilled in the art and may be done manually or by using any suitable equipment for this purpose. The rose hips are then dried and chopped. These steps may be performed in any order suitable. The drying step may be carried out by any suitable method including air-drying and/or sun-drying. The drying of the rose hips is performed under controlled and standardised temperature thus preserving the active ingredients of the rose hips and to a standardised water content. The rose hips are chopped into smaller pieces. Any suitable method may be used for chopping the rose hips. The chopped rose hips are then separated in such a way that the shells are isolated. The shells may also be termed peels. The fruit flesh, the seeds, the fine hairs found inside rose hips etc. are all discarded. The separation may be carried out using any suitable techniques. The isolated shells are then ground in a grinding mill.

The present inventors have found it to be essential that the grinding of the rose hip shells is carried out at a temperature below ambient temperature in order to preserve the active ingredients. The present inventors further found that a specific grinding mill may be required in order to comply with this requirement. In a final step the ground rose hip shells are sieved in order to obtain a particle size below 1 mm.

The particle size may be ensured by conventional means such as sieving. Any suitable method for sieving the particles may be applied. The ground rose hip particles preferably do not exceed the size of 1 mm. The grinding of the rose hip to such particle size provides a suitable form of the composition for administration. The smaller particle size further ensures efficient mixing of rose hip with vitamin C. In an embodiment the particle size is below 800 microns, preferably below 700 microns, more preferably below 600 microns, and even more preferably below 550 microns.

The process may comprise additional steps, which will be known to a person skilled in the art. Such steps may include cleaning of the rose hips to remove foreign bodies, visual inspection, control and sorting, packing, storage, transport, encapsulation etc. In an embodiment the process comprises an additional step (f), wherein particles larger than the required particle size are recycled to the grinding mill in order to improve the overall yield. In another embodiment an additional step of separation (g) is applied in which the ground particles below a specific size are sorted out. The minimum threshold limit for the particle size may be 0 microns. Thus, the particle size of the final rose hip complex then becomes from about 0 microns to about 1 mm, such as in the range of from about 150 to about 800 microns, or from 200 to about 700 microns, or from about 300 to about 700 microns, or from about 400 to about 600 microns, or from about 500 to about 600 microns, such as 550 micron. Such particle size is found particular suitable for capsules.

The particles below the minimum threshold limit may still be used for alternative purposes, e.g. for a powder product. Sieving and recycling provides for an even distribution of the particle size of the product. The process may comprise a further step (h) of mixing the dried rose hip with vitamin C. The present inventors have studied the above-mentioned composition in great detail and have identified the active ingredients of rose hip powder, which are believed to account for the beneficial effects provided by the dried rose hip. However, the present invention should not be bound by any specific theory as to explaining the biochemical and physiological effects of the composition on the human or animal body.

In an embodiment the composition obtainable according to the process of the invention comprises vitamin C, carotenes, flavonoids, triterpenoic acids, and galactolipids. In a more preferred embodiment the composition is further characterised in that vitamin C is present in an amount of from about 3.2 to about 11.2 g/kg, the carotenes are present in an amount of from about 150 to about 560 mg/kg, the flavonoids are present in an amount of from about 100 to about 1300 mg/kg, the triterpenoic acids are present in an amount of from about 500 to about 2000 mg/kg, and the galactolipids are present in an amount of from about 50 to about 500 mg/kg.

In another embodiment the composition is further characterised in that tocopherols are present in an amount of less than 120 mg/kg, such as less than 110 mg/kg, 100 mg/kg, 50 mg/kg, 10 mg/kg, or 1 mg/kg, such as from about 0.001 to about 1 mg/kg, and α-linolenic acid is present in an amount of less than 0.15 g/kg, such as less than 0.10 g/kg, 0.01 g/kg, 0.001 g/kg, 0.0001 g/kg, or 0.00001 g/kg, such as from about 0.000001 to about 0.00001 g/kg. It follows that in a preferred embodiment the composition is obtained according to the process of the present invention, i.e. the composition is derived from rose hip shells. Being that rose hip is a natural product the resulting composition of the dried rose hip may vary accordingly. In particular, the present inventors have found that the composition obtainable by the process may comprise only minute remnants of tocopherols and α-linolenic acid. Tocopherols and α-linolenic acid may be added to the composition, however, in an embodiment of the present invention the natural amounts of tocopherols and α-linolenic resulting from the process is maintained. In another embodiment tocopherols and α-linolenic acid are added to the composition.

Another aspect relates to a complex at least comprising carotenes, tocopherols, flavonoids, triterpenoic acids, α-linolenic acid, and galactolipids, and in a preferred embodiment the complex at least comprises vitamin C in an amount of from about 3.2 to about 11.2 g/kg, carotenes in an amount of from about 150 to about 560 mg/kg, tocopherols in an amount of from about 120 to about 250 mg/kg, flavonoids in an amount of from about 500 to about 1300 mg/kg, triterpenoic acids in an amount of from about 500 to about 1060 mg/kg, α-linolenic acid in an amount of from about 0.15 to about 2.2 g/kg, and galactolipids in an amount of from about 85 to about 1030 mg/kg.

In another embodiment the complex comprises vitamin C in an amount above 5 g/kg, such as 6 g/kg, preferably in an amount above 7 g/kg, more preferably above 8 g/kg, such as 9 g/kg, and even more preferably above 10 g/kg. In another embodiment the complex comprises carotenes in an amount above 200 mg/kg, preferably in an amount above 300 mg/kg, more preferably above 400 mg/kg, and even more preferably above 500 mg/kg. In yet another embodiment the complex comprises tocopherols in an amount above 150 mg/kg, preferably in an amount above 180 mg/kg, more preferably above 200 mg/kg, and even more preferably above 230 mg/kg. In another embodiment the complex comprises flavonoids in an amount above 600 mg/kg, such as 700 mg/kg, preferably in an amount above 800 mg/kg, more preferably above 900 mg/kg, and even more preferably above 1000 mg/kg. Triterpenoic acids in an amount of from about 500 to about 1060 mg/kg, In another embodiment the amount of triterpenoic acids are above 500 mg/kg, such as above 600 mg/kg or above 700 mg/kg, preferably in an amount above 800 mg/kg, more preferably above 900 mg/kg. In yet another embodiment the complex comprises α-linolenic in an amount above 0.5 g/kg, preferably in an amount above 1.0 g/kg, more preferably above 1.5 g/kg, and even more preferably above 2.0 g/kg. In another embodiment the complex comprises galactolipids in an amount above 100 mg/kg, 200 mg/kg, or 300 mg/kg, such as above 400 mg/kg, preferably in an amount above 500 mg/kg, more preferably above 600 mg/kg, such as 700 mg/kg, even more preferably above 800 mg/kg, such as 900 mg/kg, and yet even more preferably above 1000 mg/kg.

The carotenes are typically denoted with the chemical formula C₄₀H₅₆ and belong to the carotenoid family. It is organic compounds, which occur as orange-yellow to red pigments in many plants and in animal tissue, e.g. as β-carotene in carrots. Carotenes may be found in various isomeric forms and the hydrocarbon chains may be saturated or unsaturated. Lycopene is a carotene typically found in fruits and vegetables. The tocopherols of the complex are a group of closely related, fat-soluble alcohols constituting vitamin E and similar compounds, such as α-tocopherol and γ-tocopherol. Flavonoids are any of a large class of plant pigments. Flavonoids in rose hips include rutin, catechin, and quercetin and are typically denoted with the chemical formula C₁₅H₁₄O₆. The triterpenoic acids are derived from triterpene molecules and include betulinic acid, oleanolic, and ursolic acids. The triterpenoic acids are typically denoted with the chemical formula C₃₀H₄₈O₃. α-linolenic acid is a common carboxylic fatty acid and is typically denoted with the chemical formula C₁₈H₃₀O₂. The galactolipids may be any suitable galactolipid or mixture thereof such as 3-beta-D-galactopyranosyloxy-2-(octadeca- 9Z, 12Z, 15Z-trienoyloxy)propanyl octadeca-9Z,12Z,15Z-trienoate. However, the present invention is not limited to a complex consisting only of these constituents.The complex is at least obtainable by the process according to the present invention and is believed to be the active ingredient in rose hips for the treatment and prevention of inflammatory diseases. It is envisaged that the complex may be provided from other sources such as isolating and mixing the individual constituents together.

Through the identification of the above-mentioned active ingredients the present inventors have provided the means for optimising nutraceutical and pharmaceutical formulations. In particular, the present inventors have found that a high content of the active ingredients is found in the shells of rose hips and more particular preserved when prepared according to the novel process of the invention.

In one embodiment the rose hip composition is for use as a medicament. By the term "medicament" is meant that the composition is used for treating and/or inhibiting and/or preventing and/or alleviating the symptoms of a disease and/or a condition. The composition may be classified as a pharmaceutical or a nutraceutical or a dietary supplement. The composition may be formulated for any suitable form of administration to a subject.

In a preferred embodiment the composition is prepared for oral administration. The form in which the composition is prepared may be any suitable form for oral administration such as a liquid, a gel, a powder, a tablet, or a capsule. In a preferred embodiment the composition is in the form of a dry powder encapsulated in a hard shell capsule. In another embodiment it may also be administered as a powder solubilised in water.

The terms "conventional", "original", or "ordinary" in conjunction with rose hip powder/compositions may be used interchangeably and refer to products obtained by previously known methods such as the methods described in US 6,024,960 and WO 2008/006589. Rose hip powder prepared from e.g. the fruit flesh of dried rose hips and/or prepared by grinding the rose hip at a temperature above the temperature limits according to the present invention.

In particular, what is meant by conventional or original rose hip powder is products which do not comprise the composition and active ingredients according to the present invention in the concentrations described and effects documented herein.

In a preferred embodiment the rose hip composition is for use in a method for the maintenance of healthy, flexible joints, joint mobility and decreased inflammation. In such an embodiment the composition could be classified as a natural nutraceutical and/or as a dietary supplement. In another embodiment the rose hip composition is for use in the treatment and/or prevention of inflammatory diseases. Thus, the present disclosure also relates to the use of the composition for the manufacture of a medicament of the treatment and/or prevention of inflammatory diseases. The term "Inflammatory disease" is meant to include any diseases, disorders and/or conditions which are characterised by a state of inflammation in the human or animal body such as acute or chronic inflammation resulting from an excessive production of inflammatory mediators. In one embodiment the inflammatory disease is arthritis. Arthritis is a group of conditions involving damage to the joints of the body. The most common form of arthritis, osteoarthritis is a result of trauma to the joint, infection of the joint, or age. In an embodiment the arthritis is degenerative arthritis. The terms "degenerative arthritis", "osteoarthritis", and "degenerative joint disease" may be used interchangeably and refer to a clinical syndrome in which low-grade inflammation results in pain in the joints, caused by abnormal wearing of the cartilage that covers and acts as a cushion inside joints and destruction or decrease of synovial fluid that lubricates those joints. As the bone surfaces become less well-protected by cartilage, the patient experiences pain upon bearing weight, including walking and standing. Due to decreased movement because of the pain, regional muscles may atrophy, and ligaments may become more lax. In another embodiment the inflammatory disease is a rheumatic disease. Rheumatic diseases encompass a group of diseases that affect the muscular-skeletal and connective tissues of the body. These diseases are characterized by chronic inflammation that often leads to permanent tissue damage, deformity, atrophy and disability. Rheumatic diseases affect the joints, bone, soft tissue, or spinal cord and are classified as inflammatory rheumatism, degenerative rheumatism, extra-articular rheumatism, or collagen diseases. Some rheumatic diseases are known to be autoimmune diseases caused by a subject's altered immune response. In a particular embodiment of the present invention the rheumatic disease is rheumatoid arthritis. Rheumatoid arthritis is a progressive rheumatic disease. This disease is characterized by persistent inflammatory synovitis that causes destruction of cartilage and bone erosion, leading to structural deformities in the peripheral joints. The symptoms associated with rheumatoid arthritis include joint swelling, joint tenderness, inflammation, morning stiffness, and pain, especially upon flexing.

The amount of active ingredients and constituents of the complex may be quantified by metabolomic profiling. Such methods may comprise any suitable method for qualitatively and/or semi-quantitatively analysing the composition, such as mass spectrometry or chromatography. A person skilled in the art will readily recognise the methods required.

By the term "rose hip composition" is meant a composition comprising the active ingredients of rose hip, said active ingredients may also be denoted a complex as it consists of multiple constituents, which are/have been difficult to identify, understand and/or analyse. The term should not be construed as a chemical compound in which molecules, groups, or ions are attached to a central atom by coordinate bonds. The inventors have found no evidence of such chemical relationships between the constituents and are presently not aware of the inner structure of the composition. The inventors have found that the rose hip composition comprises the active ingredients in high concentrations when obtained by the process according to the present invention.

In another aspect the present disclosure relates to a rose hip powder product obtainable by the process the present invention. In an embodiment the rose hip powder product is a nutraceutical for oral administration such as a liquid, a gel, a powder, a tablet, or a capsule. In a preferred embodiment the rose hip powder product is in the form of a dry powder encapsulated in a hard shell capsule. In an evenly preferred embodiment the rose hip powder product is in the form of a powder drink.

In the following the effect of the compositions, complexes and rose hips powders according to the aspects of the invention has been evaluated. However, the invention should not be limited here to.

### Examples

### Example 1

### Anti-inflammatory properties of two rose hip preparations

In this study, the effects of two RH preparations (RH-H and RH-P) were evaluated on the production of inflammatory mediators by peripheral blood leukocytes. The preparations were prepared according to the present invention and differed only by the type of rose hips used. The preparations were tested in peripheral blood leukocytes (PBL), which were stimulated with endotoxin (lipopolysaccharide, LPS) and IFN-γ to induce an inflammatory response.

### Material and methods

*Reagents:* Two preparations of RH (RH-H and RH-P) were obtained. Compounds were dissolved in DMSO, sonicated and added to the culture medium concomitantly with the stimulus. Final DMSO concentration in culture medium was 0.5%. LPS was from *E. coli* (serotype 055:B5;Sigma, Saint-Louis, MO, USA). RPMI 1640, 2-mercaptoethanol, MEM non-essential amino acids (NEAA), phosphate buffered saline (PBS) were from Invitrogen (Carls-bad, CA, USA); fetal bovine serum (FBS) was from Sigma. Human recombinant interferon-γ (IFN-γ) was from PeproTech EC (London, UK).

*Cell culture:* Peripheral blood leukocytes (PBL) were isolated from buffy coat obtained from healthy donor at the local blood donor center (University Hospital, Basel, Switzerland). Residual erythrocytes were removed by the Dextran sedimentation procedure. PBL (at 8x106 cells/mL) were cultured in phenol red free RPMI 1640 medium, supplemented with 0.25% FBS, 0.1 mM NEAA, 50 U/mL penicillin, 50 µg/mL streptomycin and 5x10-5 M 2-mercaptoethanol. PBL were stimulated with LPS (100 ng/mL) and IFN-γ (20 U/mL) for 12 and 24 hours. Cells were lysed in RLT buffer (Qiagen) after 12 hours of culture and total RNA was further extracted.

*Multiparametric analysis of chemokines and interleukins:* Multiparametric kits were obtained from BIO-RAD Laboratories (Hercules, CA, USA) and used in the LiquiChip Workstation IS 200 (Qiagen, Hilden, Germany) according to the manufacturers' instructions. In this study were used a selection of 4 cytokines/chemokines (IL-1alpha, IL-1beta, CCL5/RANTES, CXCL10/IP-10). The data were acquired with the Luminex IS 2.3 software and evaluated with the LiquiChip Analyser software provided by Qiagen.

### Results and discussion

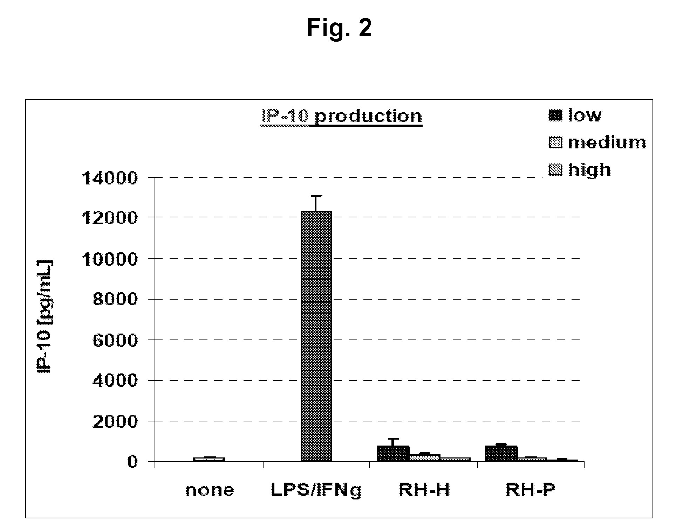

*Effects of substances on the production and secretion of two chemokines:* Peripheral blood leukocytes (PBL) were activated with LPS/IFN-γ and cultured without or with different concentrations of substances (i.e. 125 mg/L, 250 mg/L, 500 mg/L). PBL responded to LPS/IFN-γ stimulation by a markedly increased production of proinflammatory chemokines (CCL5/RANTES, CXCL10/IP-10) (Table 1 and Figure 1 and Figure 2); this is best reflected in the production ratio between stimulated and unstimulated cells (Table 1). CXCL10/IP-10 production was drastically reduced at all tested concentration. Similarly, CCL5/RANTES production was significantly and dose-dependently impaired. Collectively, RH-H and RH-P robustly modulated the secretion of chemokines. RH-H and RH-P did not substantially differ in their biological activity, as investigated by the two biological parameters.

*Effects of substances on gene expression in PBL:* The effect of substances was also evaluated at the level of gene expression. To this aim, cells were cultured at the appropriate conditions for 12 hours and the mRNA levels for 4 genes of the inflammatory pathways determined by quantitative real time RT-PCR. The observed effects are summarized in Table 2 and Figure 3. CXCL10/IP-10 was drastically inhibited by >70% at all doses tested. At different concentrations of RH (125 - 500 mg/L), the dose-dependent effects of RH-H and RH-P on modulated genes were obvious (Figure 3). Qualitatively, RH-H and RH-P exhibited similar anti-inflammatory effects on PBL. This is best illustrated by the comparable effects on the reduction of chemokines (*e.g.* CCL5/RANTES, CXCL10/IP-10). Of particular interest is the observation that RH seems to act at different physiological levels in PBL: it interferes with CXCL10/IP-10 at the transcriptional level, while it reduces CCL5/RANTES only at the posttranscriptional level and presumably acts on protein synthesis.

**Table 1**

| **Secretion of proteins by stimulated PBL** | | | | | | |
|---|---|---|---|---|---|---|
| **Protein** | **Ratio stim/ unstim** | **LPS/IFN-γ** | **LPS/IFN-γ + RH-H (500 mg/L)** | | **LPS/IFN-γ + RH-P (500 mg/L)** | |
| | | pg/mL ± SD | pg/mL ± SD | *p* value | pg/mL ± SD | *p* value |
| CCL5/RANTES | 190 | 2723 ± 758 | 1366 ± 135 | *0.04* | 833 ± 44 | *0.01* |
| CXCL10/IP-10 | 13 | 12333 ± 731 | 161 ± 32 | *<0.001* | 82 ± 22 | *<0.001* |

**Table 2**

| **Effects of substances on gene expression in stimulated PBL** | | | | | |
|---|---|---|---|---|---|
| **Gene** | **LPS/IFN-γ** | **LPS/IFN-γ + RH-H (500 mg/L)** | | **LPS/IFN-γ + RH-P (500 mg/L)** | |
| | fold change | fold change | *p* value | fold change | *p* value |
| CXCL10/IP-10 | 72.8 | 7.7 | <0.001 | 6.4 | <0.001 |

### Example 2

### Comparing different preparations and doses of rose hip powder in patients with osteoarthritis of the knee: an exploratory randomized active-controlled trial

A nutraceutical that has shown promising results in reducing pain in osteoarthritis patients is specialized rosehip powder. The dose that has been shown effective in clinical trials is 6 capsules per day. Thus, it is desirable to increase the potency of rosehips in order to improve the clinical outcome. The objective was to explore similarities and differences in the rosehip product previously shown as effective (specialized rosehip powder) said rosehip being produced according to the prior art method described in US 6,024,960 and two different doses of compositions of rosehip powder according to the invention denoted ROSENOIDS® in patients with knee osteoarthritis.

This was a 12-week single-center, patient and outcome assessor blind, three-group, exploratory randomized active-controlled trial in patients with symptomatic knee osteoarthritis. Patients were stratified by sex, and randomly assigned to receive 'Original rosehip powder' 4500 mg (n=49) group A, 'Enhanced rosehip powder' 4500 mg containing ROSENOIDS®, i.e. the composition according to the present invention (n=50) group B, or only 2250 mg rosehip powder containing ROSENOIDS®, i.e. the composition according to the present invention (n=51) group C. The primary outcome measure (after 12 weeks) was the change in the Knee injury and Osteoarthritis Outcome Score (KOOS)-item 'Pain during walking on flat surface' with individual items graded on a 5-point Likert scale from 0 to 4. Secondary outcomes included all five KOOS subscales (Function, Quality of life, Pain, Function in sport and recreation, and Symptoms).

All statistical analyses were based on the Intention-to-Treat (ITT) population (N=150), replacing missing data with a last-observation-carried-forward (LOCF)-imputation.

At baseline, the study patients had moderately severe osteoarthritis symptoms (mean 'Symptom score' 60.9 KOOS-points); the 'Pain during walking on flat surface' corresponded to a mean 1.3 KOOS item-points (0-4). During the trial period the attrition rate was equal across groups (8% group A, 6% group B, and 8% Group C, respectively). For the primary outcome, the pain relief was equally good in all groups, although the interaction between time and group indicated some differences over time (timeXgroup, P=0.075).

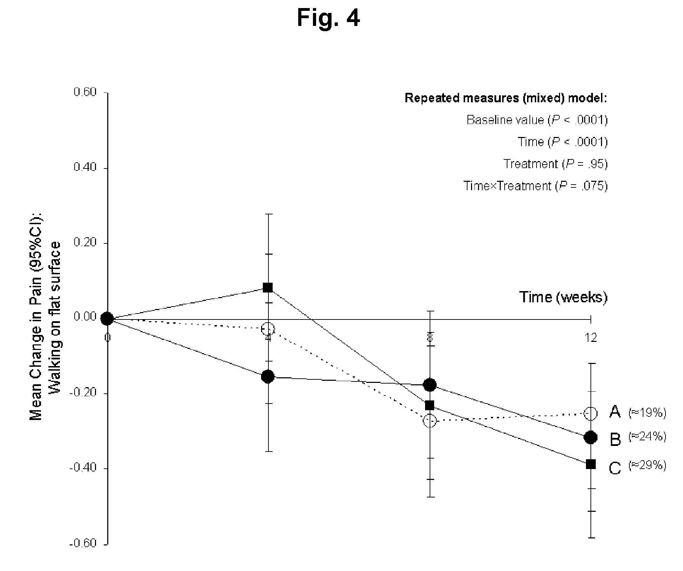

The statistically significant change in 'Pain on walking' in each group corresponded to an average improvement of 19% Group A, 24% Group B, and 29% Group C, respectively as shown in Figure 4.

Secondarily, data on KOOS-symptoms also supported low-dose enhanced rosehip powder containing ROSENOIDS® (2250 mg) compared to the 'Specialized rosehip powder' 5.97 (95%Cl: 0.92 to 11.02) KOOS-points (P=0.02).This suggests that the composition according to the present invention is at least as good as the prior art composition, even in the low dose, as the rosehip product previously used in clinical trials. It is suggested that the novel compositions according to the invention, denoted ROSENOIDS® complex accounts for the observed increased anti-inflammatory effects.

### Example 3

### Metabolomic profiling of rose hip powder samples and comparison with conventional rose hip powder

Table 3 presents samples of rose hip powder analysed by metabolomic profiling. All samples are manufactured from rose hips of the species Rosa canina (commonly known as the Dog rose), Rosa moschata, and Rosa rubiginosa. Samples A and C are rose hip powders manufactured by conventional processes, whereas samples B and D are manufactured according to the process of the present invention.

**Table 3**

| **Analysed samples** | | |
|---|---|---|
| **Identification of sample by customer:** | **Laboratory identification code:** | **Date of production:** |
| Specialized rose hip powder Sample A, Batch 201002 | LN 8271 | 17.5.2011 |
| Enhanced Specialized rose hip shell powder Sample B, Batch 201003 | LN 8272 | 17.5.2011 |
| Specialized rose hip shell powder Sample C, Batch 201004 | LN 8273 | 17.5.2011 |
| Enhanced Specialized rose hip shell powder Sample D, Batch 221011 | LN 8343 | 16.8.2011 |

### Materials and methods

### Chemicals

- n-Hexane, for gas chromatography, Merck KGaA, Germany
- Methanol, gradient grade for liquid chromatography, Merck KGaA, Germany
- Ethyl Acetate Pestanal®, Fluka Analytical, Germany
- Water (purified with a Milli-Q purification system Millipore, Eschborn, Germany)
- Polyethylene glycol (average relative molecular weight 600), Sigma-Aldrich, Steinheim, Germany

### Sample preparation

1.00 g of each sample was placed into a 15-mL plastic cuvette and shaken automatically for 5 min with:
a) 5 mL of n-hexane (for extraction of nonpolar compounds)
b) 8 mL of MeOH-H2O mixture (1:1, v/v) (for extraction polar of compounds)
c) 8 mL of H2O; supernatant was extracted by 3 mL of ethyl acetate (for isolation of flavonoids).

The mixtures obtained after shaking were separated by centrifugation (5 min, 11000 rpm) and supernatants were placed into amber vials.

### Instrumentation and measurement conditions

For the experiments a DART-TOFMS system consisting of a DART ion source (lonSense, Saugus, MA, USA), an AccuTOF LP high-resolution time-of-flight mass spectrometer [JEOL (Europe), SAS, Croissy sur Seine, France] and a HTC PAL autosampler AutoDART-96 (Leap Technologies, Carrboro, NC, USA) was used. Helium was used as an ionisation gas.

The operating conditions of the DART ion source are summarized in Table 4. Sample insertion was carried out automatically using Dip-it™ samplers (IonSense, Saugus, MA, USA). The sampling glass rod was immersed for 1 s into the sample hole of a deepwell micro-plate (Life Systems Design, Merenschwand, Switzerland) and transferred to the optimized position in front of the DART gun exit. The sample was then desorbed from the glass rod surface within 15 s, while the spectral data were recorded. To perform a mass drift compensation for accurate mass measurements and elemental composition calculations a polyethylene glycol 500 µg mL⁻¹ solution in methanol was inserted manually at the end of each analysis run. Each sample was measured in both positive and negative ion mode at temperature 250°C.

**Table 4**

| **The operating conditions of the DART - TOFMS system** | | |
|---|---|---|
| **DART parameters:** | **Positive ion mode** | **Negative ion mode** |
| Ionization gas temperature | 250 °C | 250 °C |
| Ionization gas flow | 3 l/min | 3 l/min |
| Grid electrode voltage | +250 V | -350 V |
| Desorption time | 15s | 15s |

| **TOF-MS parameter:** | **Positive ion mode** | **Negative ion mode** |
|---|---|---|
| Cone voltage | +20 V | -20 V |
| Peak voltage | 800 V | 800 V |
| Detector voltage | -2500 V | +2500 V |
| Acquisition rate | 2.5 spectra s-1 | 2.5 spectra s-1 |
| Monitored mass range *m*/*z* | 50-1000 | 50-1000 |
| Resolving power: | approx. 6000 FWHM | approx. 6000 FWHM |

The Mass Center software version 1.3 (2006) (JEOL, Tokyo, Japan) was used for data processing. Mass spectral data were obtained by averaging of the mass spectra recorded during the exposure of the sample to the DART gas beam; background ions were subtracted and a mass drift correction was performed.

### Results and discussion

### Comparison of metabolomic profiles measured in positive mode

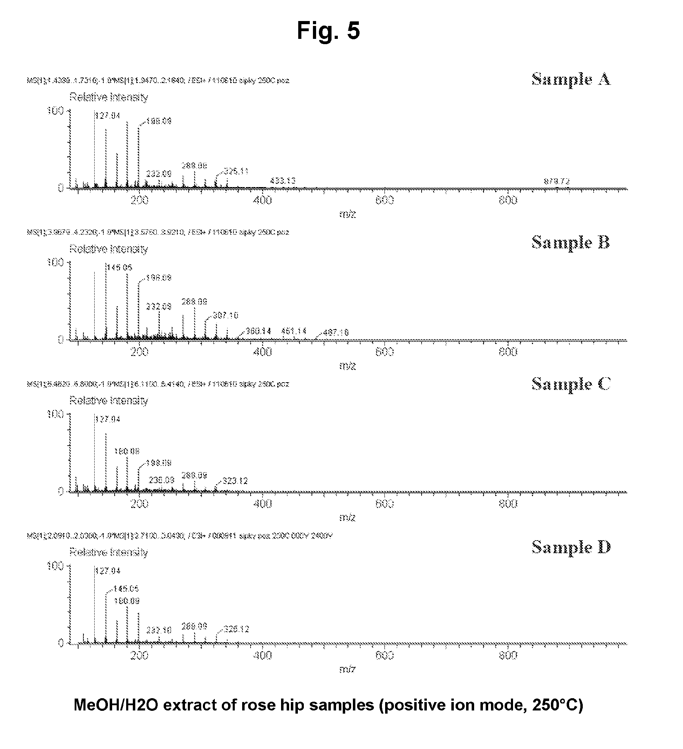

DART-TOFMS spectra of individual samples are shown in Figures 5 to 7. Biologically active compounds found in rose hip powder samples are summarized in Table 5.

**Table 5**

| **Biologically active compounds identified in positive spectra** | | | |
|---|---|---|---|
| **Compound** | **Molecular formula** | **Measured ion** | **Exact mass m/z** |
| Catechin/Epicatechin | C15H14O6 | [M+H]+ | 291.0869 |
| Quercetin | C15H10O7 | [M+H]+ | 303.0505 |
| Taxifolin | C15H12O7 | [M+H]+ | 305.0661 |
| β-sitosterol | C29H50O | [M+H-H2O]+ | 397.3834 |
| Triterpenoic acids (Betulinic, oleanolic and ursolic acid) | C30H48O3 | [M+H-H2O]+ | 439.3576 |
| β-carotene/Lycopene | C40H56 | [M+H]+ | 537.4460 |
| Linolenic acid | C18H30O2 | [M+H]+ | 279.2324 |

Spectra of the MeOH/H₂O extracts (which contain polar compounds) of all four samples are very similar and contain the same main ions (Figure 5).

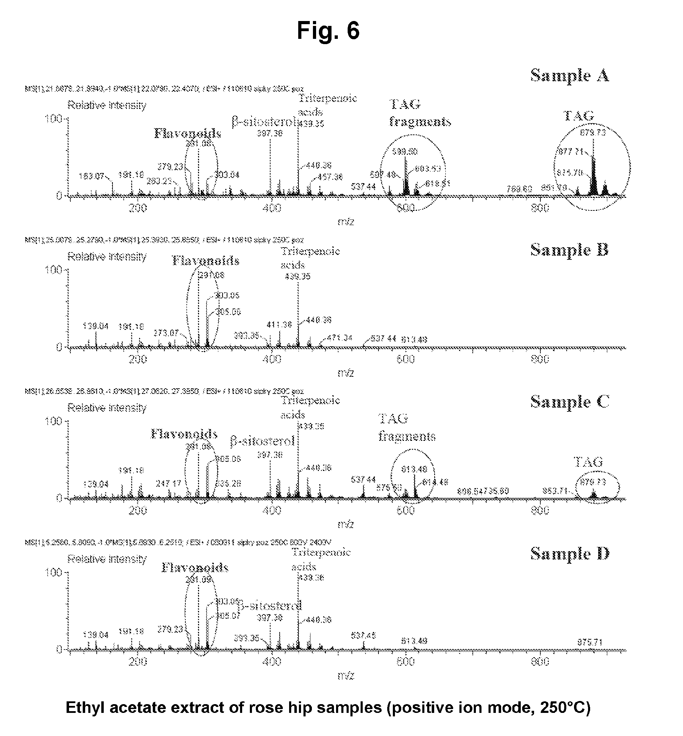

In the ethyl acetate extract, mainly flavonoids (catechin/epicatechin, quercetin and taxifolin) and non polar compounds were found (Figure 6). Tri-acylglycerols (TAGs) (m/z 850 - 920) and their fragments (m/z 570 - 640) are dominating in the spectrum of sample A, and also can be found in the spectrum of sample C. In the spectra of sample B and D, only traces of triacylglycerol ions can be found. In all of these spectra, a large peak of triterpenoic acid ion was found. A quite large peak of β-sitosterol was found in the spectra of sample A and C. Sample B seems to have the highest proportion of flavonoids compared with other substances. Generally, spectra of samples B and D are very similar.

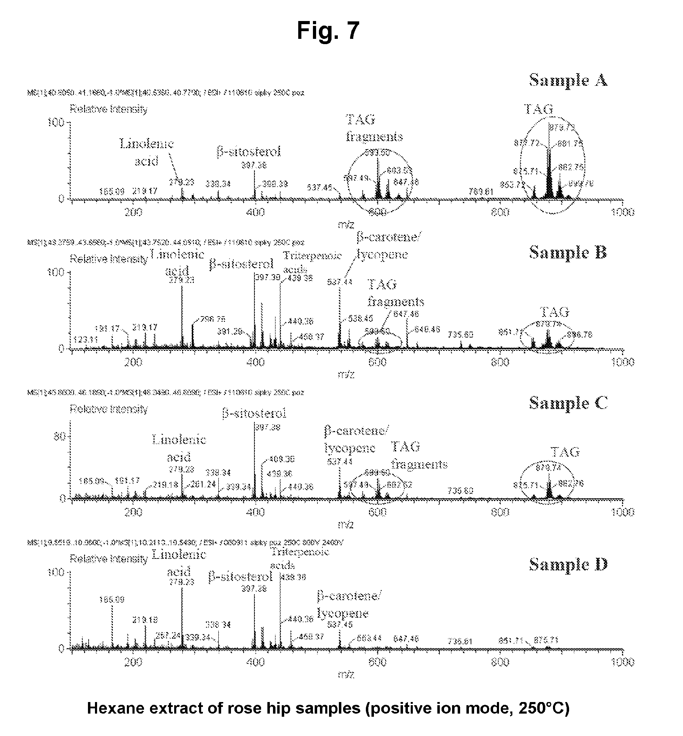

In the hexane extract, the following non polar compounds were found: linolenic acid, β-sitosterol and triterpenoic acids in each sample, β-carotene / lycopene had a large peak only in the spectra of sample B and C, and smaller peak in the spectrum of sample D. The spectra of sample B and D show higher triterpenoic acid to β-sitosterol ratio. In the spectrum of sample D, only traces of triacylglycerols were found. The hexane extract spectra are shown in Figure 7.

### Comparison of metabolomic profiles measured in negative mode

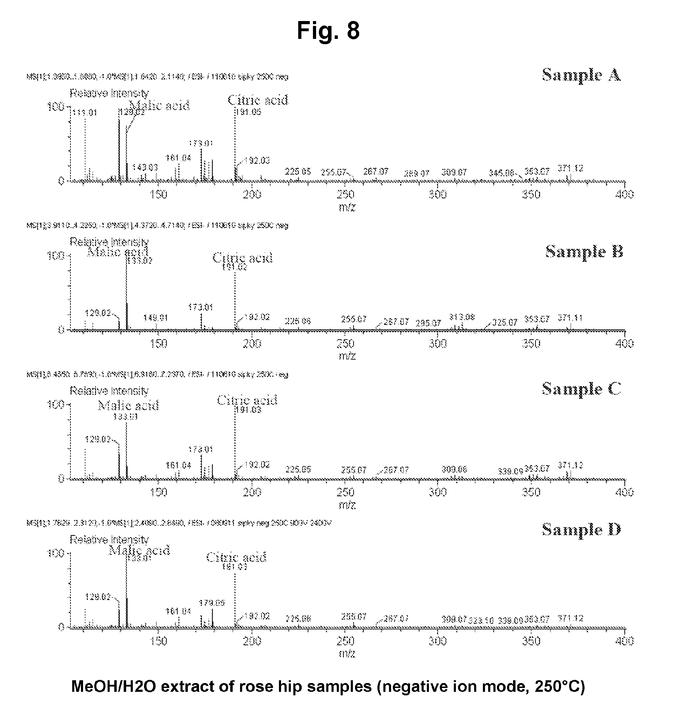

DART-TOFMS spectra of individual samples are shown in the Figures 8 to 10. Biologically active compounds found in rose hip powder samples are summarized in Table 6.

**Table 6**

| **Biologically active compounds identified in negative spectra** | | | |
|---|---|---|---|
| **Compound** | **Molecular formula** | **Measured ion** | **Exact mass** |
| Malic acid | C4H6O5 | [M-H]- | 133.0137 |
| Tartaric acid | C4H6O6 | [M-H]- | 149.0086 |
| Dehydroascorbic acid | C6H6O6 | [M-H]- | 173.0086 |
| Ascorbic acid | C6H8O6 | [M-H]- | 175.0243 |
| Citric acid | C6H8O7 | [M-H]- | 191.0192 |
| Linolenic acid | C18H30O2 | [M-H]- | 277.2168 |
| Linoleic acid | C18H32O2 | [M-H]- | 279.2324 |
| Catechin/Epicatechin | C15H14O6 | [M-H]- | 289.0712 |
| Quercetin | C15H10O7 | [M-H]- | 301.0348 |
| Taxifolin | C15H12O7 | [M-H]- | 303.0505 |
| Triterpenoic acids | C30H48O3 | [M-H]- | 455.3525 |

In MeOH/H₂O extract polar molecules, mainly organic acids (malic acid, tartaric acid and citric acid), and vitamin C (ascorbic and dehydroascorbic acid) were found in all samples (Figure 8) and all spectra are similar.

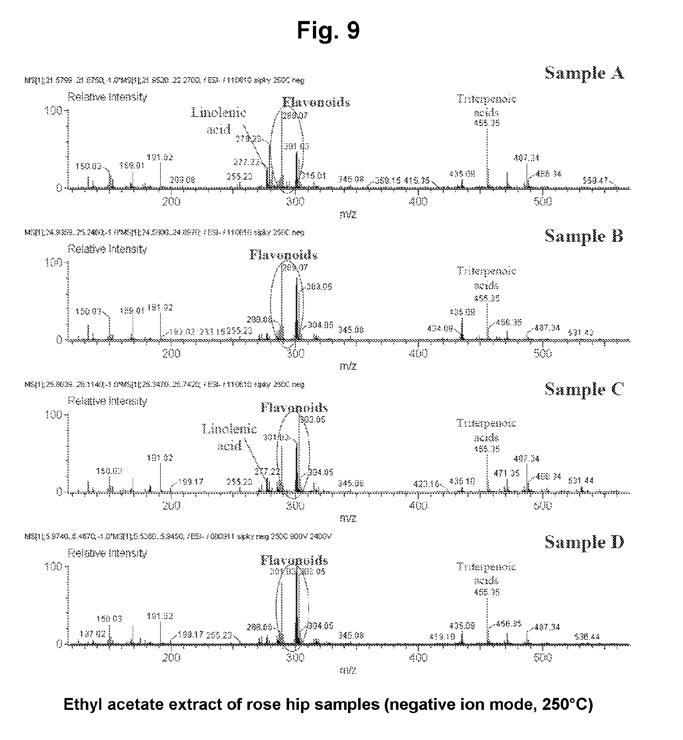

The same flavonoids were found in the ethyl acetate extract as in the positive mode. Furthermore, large peaks of triterpenoic acids were found in all of these spectra. In the spectra of sample A linolenic acid was also found (Figure 9).

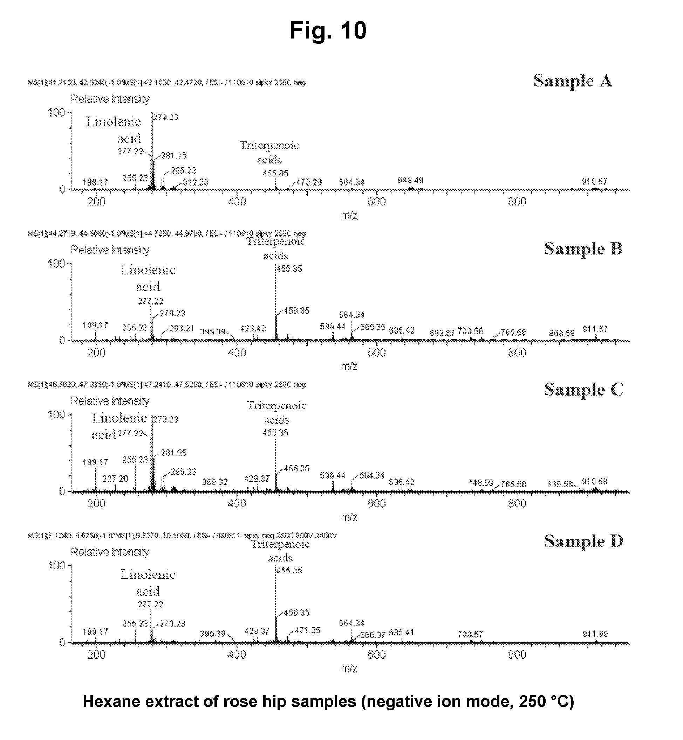

In spectra of the hexane extracts mainly fatty acids and triterpenoic acids were found. As can be seen from Figure 10, sample A has much higher intensities of fatty acid ions than triterpenoic acids ion, compared to other samples. In both of the spectra of sample B and D, the peak of triterpenoic acids was the most dominant.

### Summary of results

The main differences between these samples were in the content of triacylglycerols and fatty acids. Sample A shows the highest content of TAGs and, on the contrary, it seems that sample B and D has the lowest TAG content. These compounds can originate from seeds which can be present in the sample A and C. In sample B and D it is clear that an increased amount of flavonoids and triterpenoic acids is present.

### Example 4

### Determination of flavonoids in rose hip powders

### Materials and methods

### Chemicals

- Methanol, gradient grade for liquid chromatography, Merck KGaA, Germany
- Formic acid, p.a., 98%, Fluka analytical, Germany
- Acetonitrile, gradient grade for HPLC, ≥99.9%, Sigma-Aldrich, Germany
- Water (purified with a Milli-Q purification system Millipore, Eschborn, Germany)
- (+)-Catechin hydrate ≥98% (HPLC), powder, Sigma-Aldrich, Germany
- Quercetin ≥98% (HPLC), Sigma-Aldrich, Germany
- Rutin trihydrate ≥90% (HPLC), Sigma-Aldrich, Germany

### Sample preparation

1 g of sample was extracted with 5% solution of formic acid in methanol. Aliquot was evaporated to dryness and dissolved in acetonitrile/water (1/1, v/v) mixture. The solution was filtered through micro filter 5 µm into an amber vial.

### Instrumentation and measurement conditions

Flavonoids were determined using ultra performance liquid chromatography coupled with tandem mass spectrometer.
- Instrument: UPLC system: Acquity UPLC, Waters, USA
- Detector: AB SCIEX QTRAP® 5500, AB SCIEX, USA
- Column : Acquity BEH C18 (50 x 2.1 mm; 1.7 µm), Waters, USA
- Mobile phase:
   A. 0.6% formic acid in water
   B. 0.6% formic acid in acetonitrile
- Detection: MS/MS (ESI-)
- Detector conditions:

| **Analyte** | **Parent ion (*m*/*z*)** | **Daughter ion (*m*/*z*)** | **DP^{a} (V)** | **CE^{b} (V)** | **CXP^{c} (V)** |
|---|---|---|---|---|---|
| Rutin | 608.9 | 300.0 | -205 | -50 | -13 |
| | | 271.1 | -205 | -72 | -13 |
| | | 255.0 | -205 | -70 | -11 |
| Catechin | 288.9 | 245.0 | -145 | -22 | -11 |
| | | 108.9 | -145 | -34 | -9 |
| | | 123.1 | -145 | -40 | -9 |
| Quercetin | 300.9 | 151.1 | -115 | -30 | -7 |
| | | 179.0 | -115 | -26 | -11 |
| | | 107.0 | -115 | -38 | -5 |

| | | | | | |
|---|---|---|---|---|---|
| ^{a} Declustering potential ^{b} Collision energy ^{c} Cell exit potential | | | | | |

### Results

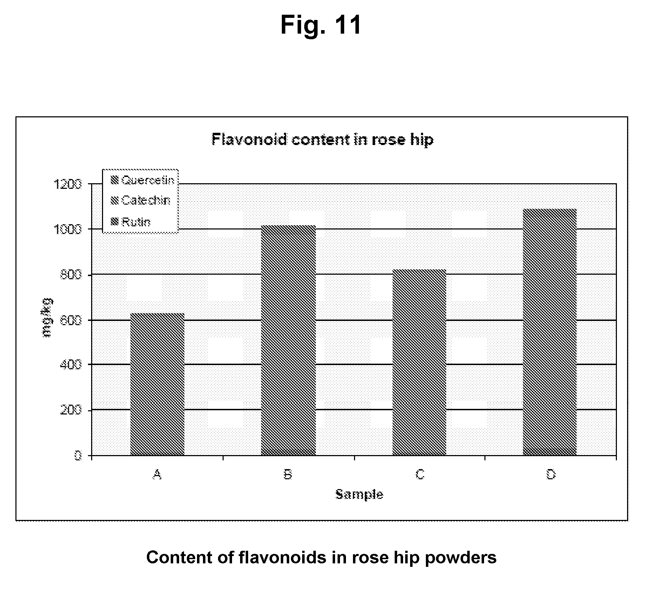

Catechin was the main of the monitored flavonoids. The highest levels of all flavonoids were found in sample D, followed by sample B. The flavonoid content is summarized in Table 7 and graphical comparison is shown in Figure 11.

**Table 7**

| **Content of flavonoids in rose hip powders** | | | | | | |
|---|---|---|---|---|---|---|
| **Sample** | **Batch** | **Laboratory identification code:** | **Rutin (mg kg-1)** | **Catechin (mg kg-1)** | **Quercetin (mg kg-1)** | **SUM of flavonoids (mg kg-1)** |
| A | 201002 | LN 8271 | 11.2 | 606.0 | 6.0 | 623.2 |
| B | 201003 | LN 8272 | 28.0 | 950.0 | 34.4 | 1012.4 |
| C | 201002 | LN 8273 | 14.8 | 798.0 | 8.8 | 821.6 |
| D | 221011 | LN 8343 | 31.0 | 1020.0 | 36.6 | 1087.6 |

## Claims

1. A process for the manufacture of dried rose hip powder comprising the steps of
• providing harvested rose hip,
• drying and chopping the rose hip,
• separating the chopped rose hip and isolating the shells,
• grinding the shells, and
• optionally sieving the ground rose hip shells to a particle size below 1 mm,
wherein cooling is applied during the grinding step so that the grinding is performed at a constant temperature, said temperature being below 40°C, preferably below 30°C, more preferred below 25°C even more preferred below 20°C.

2. The process according to claim 1, wherein the cooling applied is air cooling.

3. The process according to claim 1 or 2 further comprising the step of mixing the ground rose hip with vitamin C added in an amount from 30 mg added vitamin C per g rose hip powder to about 80 mg per g rose hip powder, such as about 40 mg added vitamin C to1 g ground rose hip powder.

4. The process according to any of the claims 1 to 3, wherein the rose hips are obtained from any one of the species of plants belonging to the *Rosa* family such as Rosa canina, Rosa mochata, Rosa rubiginosa, Rosa gallica, Rosa conditata, Rosa rugosa, Rosa hugonis, Rosa nitida, Rosa pendulina, Rosa pimpinellifolia and Rosa sericea and any mixture thereof.

## Patentansprüche

1. Verfahren zum Herstellen von getrocknetem Hagebuttenpulver, umfassend die Schritte:
• Bereitstellen geernteter Hagebutten,
• Trocknen und Zerhacken der Hagebutten,
• Trennen der zerhackten Hagebutten und Abtrennen der Schalen,
• Mahlen der Schalen, und
• optional Sieben der gemahlenen Hagebuttenschalen zu einer Partikelgröße unter 1 mm,
wobei eine Kühlung während des Mahlschrittes angewendet wird, so dass das Mahlen bei einer konstanten Temperatur durchgeführt wird, wobei die Temperatur unter 40°C, vorzugsweise unter 30°C, mehr bevorzugt unter 25°C und noch weiter bevorzugt unter 20°C ist.

2. Verfahren nach Anspruch 1, wobei die angewendete Kühlung eine Luftkühlung ist.

3. Verfahren nach Anspruch 1 oder 2, weiterhin umfassend den Schritt: Mischen der gemahlenen Hagebutten mit Vitamin C, welches in einer Menge von 30 mg hinzugesetztem Vitamin C pro g Hagebuttenpulver bis ungefähr 80 mg pro g Hagebuttenpulver hinzugefügt wird, wie ungefähr 40 mg hinzugefügtes Vitamin C zu 1 g gemahlenen Hagebuttenpulver.

4. Verfahren nach einem der Ansprüche 1 bis 3, wobei die Hagebutten von einer Spezies Pflanzen erhalten werden, welche zu der Rosenfamilie gehören, wie Rosa canina, Rosa mochata, Rosa rubiginosa, Rosa gallica, Rosa conditata, Rosa rugosa, Rosa hugonis, Rosa nitida, Rosa pendulina, Rosa pimpinellifolia und Rosa sericea und Mischungen davon.

## Revendications

1. Procédé pour la fabrication de poudre de cynorrhodon séchée, comprenant les étapes suivantes :
- fournir du cynorrhodon moissonné,
- laisser sécher et hacher le cynorrhodon,
- séparer le cynorrhodon haché et isoler les cosses,
- broyer les cosses, et
- optionnellement tamiser les cosses de cynorrhodon broyées jusqu'à obtenir une taille de particule inférieure à 1 mm,
dans lequel un refroidissement est appliqué pendant l'étape de broyage de telle sorte que le broyage soit exécuté à une température constante, ladite température étant inférieure à 40°C, de préférence inférieure à 30°C, mieux encore inférieure 25°C, et idéalement inférieure à 20°C.

2. Procédé selon la revendication 1, dans lequel le refroidissement appliqué est un refroidissement par air.

3. Procédé selon la revendication 1 ou 2, comprenant en outre l'étape de mélange du cynorrhodon broyé avec de la vitamine C ajoutée en une quantité comprise entre 30 mg de vitamine C ajoutée par gramme de poudre de cynorrhodon et environ 80 mg par gramme de poudre de cynorrhodon, par exemple environ 40 mg de vitamine C ajoutée à 1 g de de poudre de cynorrhodon.

4. Procédé selon l'une quelconque des revendications 1 à 3, dans lequel les cynhorrodons sont obtenus à partir de n'importe laquelle des espèces de plantes appartenant à la famille des rosacées (*Rosa*), telles que les Rosa canina, Rosa mochata, Rosa rubiginosa, Rosa gallica, Rosa conditata, Rosa rugosa, Rosa hugonis, Rosa nitida, Rosa pendulina, Rosa pimpinellifolia et Rosa sericea, ainsi que tout mélange de celles-ci.
